# EUROPEAN PATENT SPECIFICATION

(11) **EP 1 923 002 B1**
(45) Date of publication and mention of the grant of the patent: **02.03.2011**
(21) Application number: 07018335.5
(22) Date of filing: 19.09.2007
(51) Int. Cl.: A61B 8/00, G06F 19/00, G06Q 50/00

(54) **System and method for providing contents used in ultrasound diagnostic device**
System und Verfahren zum Bereitstellen von Inhalten zur Verwendung in Ultraschalldiagnosevorrichtungen
Système et procédé pour fournir les contenus utilisés dans un dispositif de diagnostic à ultrasons

(30) Priority: 20.11.2006 KR 20060114653
(43) Date of publication of application: 21.05.2008
(73) Proprietor: MEDISON CO., LTD., Kangwon-do 250-870 (KR)
(72) Inventor: Kim, Jae Gyoung, Seoul 135-280 (KR); Song, Young Seuk, Seoul 135-280 (KR); Ahn, Mi Jeoung, Seoul 135-280 (KR)
(74) Representative: Schmid, Wolfgang

(56) References cited:
- US-A1- 2003 153 816
- US-A1- 2007 179 804

## Description

### BACKGROUND

### 1. Field

The present invention generally relates to a system and method for providing contents to be used in an ultrasound diagnostic device.

### 2. Background

An ultrasound diagnostic device has a wide range of applications and has become one of the important diagnostic devices. Specifically, due to its non-invasive and non-destructive nature, the ultrasound diagnostic device has been extensively used in the medical profession. Modern high-performance ultrasound diagnostic devices are commonly used to produce two or three-dimensional images of internal features of an object.

Generally, an ultrasound diagnostic device comprises software for driving a tool and producing two or three-dimensional images of internal features of an object. Further, the ultrasound diagnostic device may comprise various tools for obtaining two or three-dimensional images of internal features of an object or for improving image quality of said images by modifying their image settings. These tools and software are provided by the manufacturer of the ultrasound diagnostic device or by a third party. Such tools and software can be updated with new versions.

When a new version of software is developed, the manufacturer of the ultrasound diagnostic device (or a third party) must typically visit the location where the ultrasound diagnostic device is installed (e.g., medical center) in order to install the new version to update the software included in the device. This requires a lot of time and effort.
For example, although a new version of a tool is developed to reflect various guidelines for obtaining heart images presented by relevant societies such as ASE and ACC, it is difficult to share and manage the new version.

A contents providing system according to the preamble of claim 1 and a method of providing contents by using a contents providing system according to the preamble of claim 5 are known from US 2003/153816 A1.

### BRIEF DESCRIPTION OF THE DRAWINGS

Arrangements and embodiments may be described in detail with reference to the following drawings in which like reference numerals refer to like elements and wherein:

FIG. 1 is a block diagram illustrating a system for providing contents to be used in an ultrasound diagnostic device in accordance with one embodiment of the present invention;

FIG. 2 is a flow chart showing procedures for providing contents in accordance with one embodiment of the present invention;

FIG. 3 is a flow chart showing procedures for downloading contents in accordance with one embodiment of the present invention; and

FIG. 4 is a flow chart showing procedures for uploading contents in accordance with one embodiment of the present invention.

### DETAILED DESCRIPTION OF THE PRESENT INVENTION

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure.

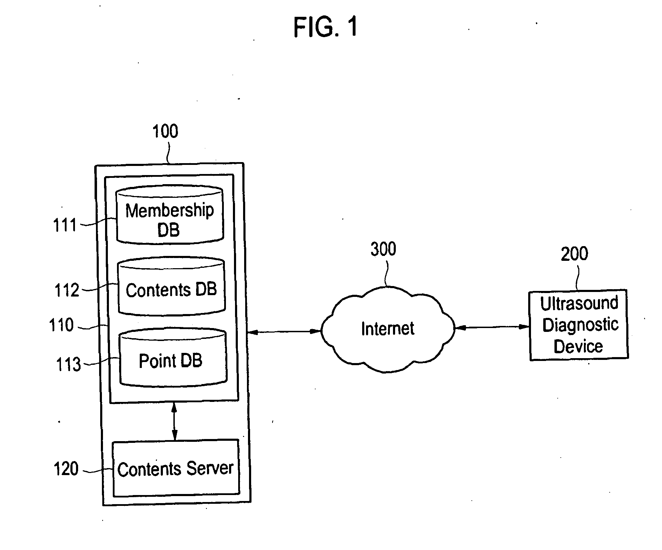

FIG. 1 illustrates a scheme for a system 100 adapted to provide contents to be used in an ultrasound diagnostic device in accordance with one embodiment of the present invention. As shown in FIG. 1, such a system 100 is connected to an ultrasound diagnostic device 200 through the Internet 300 and comprises a database 110 and a contents server 120. The Internet 300 should be interpreted and understood as a communication network wherein conventional wired and wireless communication networks are interconnected. The ultrasound diagnostic device 200 may be any device, which can obtain ultrasound images of an object.

As shown in FIG. 1, the database 110 comprises a membership database 111, a contents database 112 and a point database 113.

The membership database 111 stores information associated with members registered to the contents providing system 100 ("membership information"). In particular, the membership database 111 may store membership information including the names, addresses, phone numbers, e-mail addresses, IDs and passwords of the members using the ultrasound diagnostic device 200 and registered to the contents providing system 100.

The contents database 112 stores contents, which may be used in the ultrasound diagnostic device 200. For example, such contents may include the following: a software update to update the software for driving and operating the ultrasound diagnostic 200, as well as to obtain two or three-dimensional images of internal features of an object; a tool for setting a probe according to patient status and diagnosed parts; an image setting tool for obtaining optimal images by adjusting images according to the patient status and diagnosed parts; a measure table tool for each application; and a measure report. A measure table may include reference values for respective body parts of a fetus, which are classified according to weekly development stage.

The point database 113 stores information regarding the ID and points of each of the members.

The contents server 120 manages contents inputted to or outputted from the database 110. According to one embodiment of the present invention, the contents server 120 provides a web page for uploading and downloading contents to the ultrasound diagnostic device 200 connected to the contents providing system 100. The web page may be implemented with HTML (Hyper-Text Markup Language) and/or WMI, (Wireless Markup Language), which may be accessed by the ultrasound diagnostic device 200. The contents server 120 stores the contents uploaded from the ultrasound diagnostic device 200 in the contents database 112 and updates point data in the point database 113 by adding predetermined points to the points of the member who has uploaded the contents. The contents server 120 retrieves the contents, which the ultrasound diagnostic device 200 requests to download from the contents database 112, and obtains the contents to be downloaded to the ultrasound diagnostic device 200. Further, the contents server 120 updates point data in the point database 113 by subtracting predetermined points from the points of the member who has downloaded the contents. The contents server 120 further comprises a communication module (not shown) connected to the contents providing system 100 through the Internet 300.

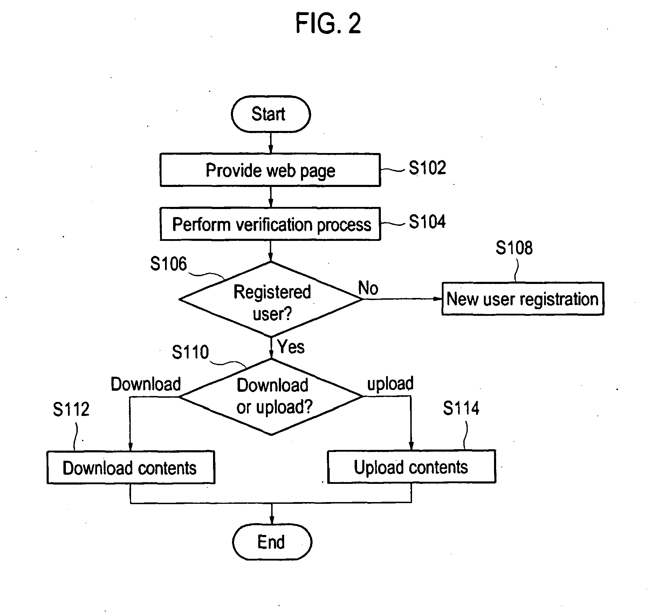

The procedures for providing contents in accordance with the embodiment of the present invention are explained below with reference to FIGS. 2-4.

As shown in FIG. 2, when the ultrasound diagnostic device connects to the contents providing system 100 through the Internet 300, the contents server 120 provides a web page for contents upload/download to the ultrasound diagnostic device 200 (S102) and then performs verification process on the ultrasound diagnostic device 200 (S104). The verification process is performed by comparing the ID and password entered into the ultrasound diagnostic device 200 with the membership information stored in the membership database 111.

The contents server 120 performs the verification process to determine whether the user of the ultrasound diagnostic device 200 is registered to the contents providing system 100 (S106). If the user of the ultrasound diagnostic device 200 is not a registered user, then the contents server 120 performs a new user registration process (S108). The new user registration process may be implemented by using well-known techniques. Thus, detailed explanations thereon are omitted herein.

If the user of the ultrasound diagnostic device 200 is a registered user, then the contents server 120 determines whether a predetermined menu arranged in the web page is selected (S110). For the sake of convenience, it is assumed that an upload menu for uploading contents to the contents providing system 100 and a download menu for downloading contents from the contents providing system 100 are included in the web page. However, one of ordinary skill in the art understands that various types of menus may be added to the web page as necessary.

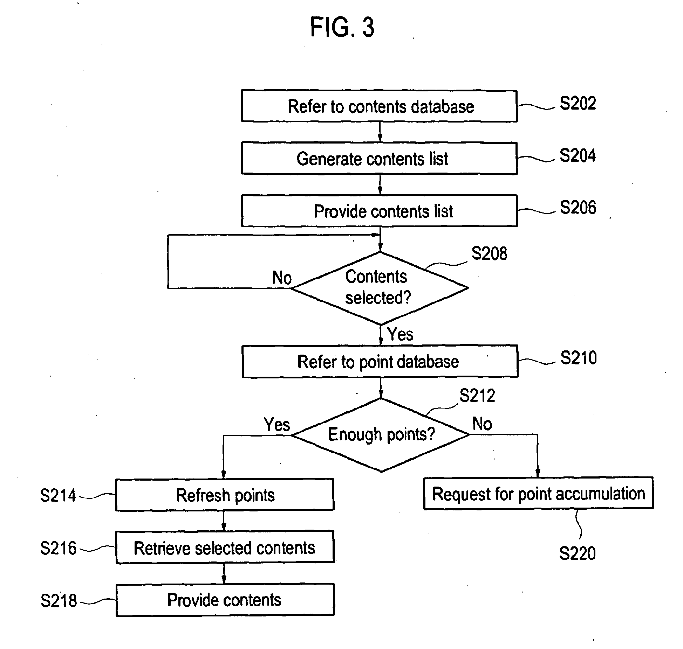

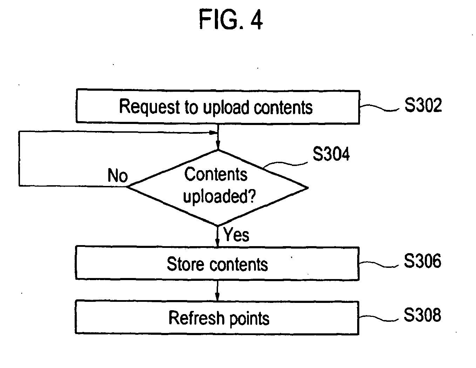

If it is determined that the download menu is selected in S110, then the contents server 120 performs a contents download process (S112). If it is determined that the upload menu is selected in S110, then the contents server 120 performs a contents upload process (S114). The steps S112 and S114 will be explained with reference to FIGS. 3 and 4.

As shown in FIG. 3, the contents server 120 refers to the contents database 112 (S202) and generates a list of contents stored in the contents database 112 (S204). Further, the contents server 120 provides the list to the ultrasound diagnostic device 200 (S206).

When some contents are selected by the ultrasound diagnostic device 200 (S208), the contents server 120 refers to the point database 113 (S210) and determines whether the user of the ultrasound diagnostic device 200 (i.e., registered member) has enough points for downloading the contents (S212).

If it is determined that the member does not have enough points for the download in S212, then the contents server 120 requests for point accumulation to the ultrasound diagnostic device 200 (S220). Such point accumulation may be performed by uploading contents or purchasing extra points for cash or credit cards. Since the point purchase for cash or credit cards may be implemented by using well-known purchase processes, detailed explanations thereon are omitted herein.

If it is determined that the member has enough points for downloading the contents in S212, then the contents server 120 reduces the points of the member who has requested to download the contents (S214). That is, the contents server 120 updates the member's points by subtracting predetermined contents download points from the points stored in the point database 113.

The contents server 120 retrieves the contents selected by the ultrasound diagnostic device 200 from the contents database 112 (S216) and provides the contents to the ultrasound diagnostic device 200. That is, the contents server obtains the contents to be downloaded to the ultrasound diagnostic device 200 through the Internet 300 (S218).

As shown in FIG. 4, the contents server 120 requests the ultrasound diagnostic device 200 to upload contents (S302). When contents are uploaded from the ultrasound diagnostic device 200 (S304), the uploaded contents are stored in the contents database 112 (S306) and predetermined points are given to the member who has uploaded the contents (S308). That is, the contents server 120 updates the member's points by adding predetermined contents upload points to the points stored in the point database 113.

In accordance with one embodiment of the present invention, there is provided a contents providing system accessible by an ultrasound diagnostic device through a communication network, which comprises: a database adapted to store a plurality of software contents; and a server operable to store first software contents uploaded from the ultrasound diagnostic device in said database, said server being responsive to a download request for second software contents from the ultrasound diagnostic device to retrieve the second software contents from said database for provision to the ultrasound diagnostic device.

In accordance with one embodiment of the present invention, there is provided a method of providing contents by using a contents providing system interconnected with an ultrasound diagnostic device through a communication network, which comprises: (a) arranging a contents database to store a plurality of software contents in the contents providing system; (b) connecting the ultrasound diagnostic device to the contents providing system through the communication network; (c) uploading first software contents from the ultrasound diagnostic device to the contents providing system to store the first software contents in the contents database; and (d) upon a download request for second software contents from the ultrasound diagnostic device, retrieving the second software contents from the contents database to provide the second software contents to the ultrasound diagnostic device.

Any reference in this specification to "one embodiment," "an embodiment," "example embodiment," etc., means that a particular feature, structure or characteristic described in connection with the embodiment is included in at least one embodiment of the present invention. The appearances of such phrases in various places in the specification are not necessarily all referring to the same embodiment. Further, when a particular feature, structure or characteristic is described in connection with any embodiment, it is submitted that it is within the purview of one skilled in the art to effect such feature, structure or characteristic in connection with other ones of the embodiments.

## Claims

1. A contents providing system (100) accessible by an ultrasound diagnostic device (200) through a communication network (300), comprising a data base (110) and a server (120),
**characterized in that:**
the database (110) is configured to store a plurality of software contents including a software update to update software for driving and operating the ultrasound diagnostic device (200), information on members registered to said contents providing system (100), and information on point data for each of the members; and
the server (120) is configured to receive first software contents for the ultrasound diagnostic device (200) which the ultrasound diagnostic device (200) is uploading and store the software contents in said database (110), said server (120) being further configured to update the point information by adding a first predetermined point to the point data for the member who has uploaded the software contents and responsive to a download request for second software contents from the ultrasound diagnostic device (200) to retrieve the second software contents from said database (110) for provision to the ultrasound diagnostic device (200), wherein the server (120) is further configured to update the point information by subtracting a second predetermined point from the point data for the member who has downloaded the second software contents.

2. The contents providing system of Claim 1, wherein said database (120) comprises:
a membership database (111) for storing the information on the members registered to said contents providing system;
a contents database (112) for storing the first and second software contents; and
a point database (113) for storing information on the point data for each of the members, which are necessary for downloading the second software contents, wherein the points are accumulated by uploading the first software contents or purchasing extra points for cash or credit cards.

3. The contents providing system of Claim 2, wherein said server (120) generates a list of the software contents stored in said contents database (112) so as to provide the same to the ultrasound diagnostic device (200); upon receiving contents selection information from the ultrasound diagnostic device (200), retrieves software contents corresponding to the contents selection information from said contents database (112) so as to provide the same to the ultrasound diagnostic device (200); and subtracts predetermined download points for downloading the software contents from the points stored in said point database (113).

4. The contents providing system of Claim 2, wherein said server (120) stores the first software contents uploaded from the ultrasound diagnostic device (200) in said contents database upon an upload request from the ultrasound diagnostic device (200) and adds predetermined upload points for uploading the software contents to the points stored in said point database (113).

5. A method of providing contents by using a contents providing system interconnected with an ultrasound diagnostic device through a communication network,
**characterized by** the steps of:
(a) arranging a contents database to store a plurality of software contents including a software update to update a software for driving and operating the ultrasound diagnostic device in the contents providing system information on members registered to said contents providing system, and information on point data for each of the members;
(b) connecting the ultrasound diagnostic device to the contents providing system through the communication network;
(c) receiving first software contents for the ultrasound diagnostic device which the ultrasound diagnostic device is uploading and storing the first software contents in the contents database, and updating the point information on the point data by adding a first predetermined point to the point data for the member who has uploaded the first software contents; and
(d) upon a download request for second software contents from the ultrasound diagnostic device, retrieving the second software contents from the contents database for provision to the ultrasound diagnostic device, updating the point information on the point data by subtracting a second predetermined point from the point data for the member who has downloaded the software contents.

6. The method of Claim 5, wherein the contents providing system comprises a point database for storing information on point data for each of members registered to the contents providing system, and wherein the step (c) comprises:
(c1) requesting the ultrasound diagnostic device to upload the first software contents;
(c2) storing the first software contents uploaded from the ultrasound diagnostic device in the contents database;
(c3) adding predetermined upload points for uploading the first software contents to the points stored in the point database.

7. The method of Claim 5, wherein the contents providing system comprises a point database for storing information on points of each of members registered to the contents providing system, and wherein the step (d) comprises:
(d1) generating a list of the software contents stored in the contents database;
(d2) providing the list to the ultrasound diagnostic device;
(d3) receiving contents selection information from the ultrasound diagnostic device;
(d4) retrieving software contents corresponding to the contents selection information from the contents database to provide the contents to the ultrasound diagnostic device; and
(d5) subtracting predetermined download points for downloading the contents from the points stored in the point database.

## Patentansprüche

1. System (100) zum Bereitstellen von Inhalten, die einer Ultraschalldiagnosevorrichtung (200) durch ein Kommunikationsnetzwerk (300) zugänglich sind, mit einer Datenbank (110) und einem Server (120), **dadurch gekennzeichnet, dass** die Datenbank (110) dafür vorgesehen ist, eine Vielzahl von Softwareinhalten einschließlich eines Software-Updates, um Software zum Antreiben und Betreiben der Ultraschalldiagnosevorrichtung (200) upzudaten bzw. zu aktualisieren, Informationen hinsichtlich an dem Inhalte Bereitstellungssystem (100) registrierten Mitgliedern und Informationen hinsichtlich Punktdaten für jedes der Mitglieder zu speichern; und
der Server (120) dafür vorgesehen ist, erste Softwareinhalte für die Ultraschalldiagnosevorrichtung (200) zu empfangen, welche die Ultraschalldiagnosevorrichtung (200) hochlädt, und die Softwareinhalte in der Datenbank (110) zu speichern, wobei der Server (120) des weiteren dafür vorgesehen ist, die Punktinformationen durch Addieren eines ersten vorbestimmten Punkts zu den Punktdaten für das Mitglied, welches die Softwareinhalte hochgeladen hat, hochzuladen und als Reaktion auf eine Downloadanfrage bezüglich zweiter Softwareinhalte von der Ultraschalldiagnosevorrichtung (200) die zweiten Softwareinhalte von der Datenbank (110) zur Bereitstellung für die Ultraschalldiagnosevorrichtung (200) abzurufen, wobei der Server (120) des weiteren dafür vorgesehen ist, die Punktinformationen durch Subtrahieren eines zweiten vorbestimmten Punkts von den Punktdaten für das Mitglied, welches die zweiten Softwareinhalte heruntergeladen hat, zu aktualisieren.

2. System zum Bereitstellen von Inhalten nach Anspruch 1, wobei die Datenbank (120) Folgendes aufweist:
eine Mitgliederdatenbank (111) zum Speichern der Informationen bezüglich der in dem System zum Bereitstellen von Inhalten registrierten Mitgliedern;
eine Inhaltsdatenbank (112) zum Speichern der ersten und zweiten Softwareinhalte; und
eine Punktdatenbank (113) zum Speichern von Informationen bezüglich der Punktdaten für jedes der Mitglieder, welche zum Herunterladen der zweiten Softwareinhalte notwendig sind, wobei die Punkte durch das Hochladen der ersten Softwareinhalte oder Kaufen von Extrapunkten für Geldautomaten- oder Kreditkarten angesammelt werden.

3. System zum Bereitstellen von Inhalten nach Anspruch 2, wobei der Server (120) eine Liste der in der Inhaltsdatenbank (112) gespeicherten Softwareinhalte erzeugt, um dieselbe für die Ultraschalldiagnosevorrichtung (200) zur Verfügung zu stellen; nach dem Empfangen der Informationen über den ausgewählten Inhalt von der Ultraschalldiagnosevorrichtung (200) Softwareinhalte abruft, welche den Informationen über den ausgewählten Inhalt von der Inhaltsdatenbank (112) entsprechen, um dieselben der Ultraschalldiagnosevorrichtung (200) zur Verfügung zu stellen; und vorbestimmte Herunterladepunkte zum Herunterladen der Softwareinhalte von den in der Punktdatenbank (113) gespeicherten Punkten subtrahiert.

4. System zum Bereitstellen von Inhalten nach Anspruch 2, wobei der Server (120) die ersten Softwareinhalte, welche von der Ultraschalldiagnosevorrichtung (200) hochgeladen wurden, in der Inhaltsdatenbank auf eine Hochladeanfrage von der Ultraschalldiagnosevorrichtung (200) speichert und vorbestimmte Hochladepunkte zum Hochladen der Softwareinhalte zu den in der Punktdatenbank (113) gespeicherten Punkten addiert.

5. Verfahren zum Bereitstellen von Inhalten unter Verwendung eines Systems zum Bereitstellen von Inhalten, welches mit einer Ultraschalldiagnosevorrichtung durch ein Kommunikationsnetzwerk verbunden ist,
**gekennzeichnet durch** die folgenden Schritte:
a) Einrichten einer Inhaltsdatenbank, um eine Vielzahl von Softwareinhalten einschließlich eines Softwareupdates zum Updaten einer Software zum Antreiben und Betreiben der Ultraschalldiagnosevorrichtung in dem System zum Bereitstellen von Inhalten, Informationen bezüglich in dem System zum Bereitstellen von Inhalten registrierten Mitgliedern und Informationen bezüglich Punktdaten für jedes der Mitglieder zu speichern;
b) Verbinden der Ultraschalldiagnosevorrichtung mit dem System zum Bereitstellen von Inhalten durch das Kommunikationsnetzwerk;
c) Empfangen von ersten Softwareinhalten für die Ultraschalldiagnosevorrichtung, welche die Ultraschalldiagnosevorrichtung hochlädt, und Speichern der ersten Softwareinhalte in der Inhaltsdatenbank und Updaten der Punktinformationen bezüglich der Punktdaten **durch** Addieren eines ersten vorbestimmten Punkts zu den Punktdaten für das Mitglied, welches die ersten Softwareinhalte hochgeladen hat; und
d) nach einer Herunterladeanfrage bezüglich zweiter Softwareinhalte von der Ultraschalldiagnosevorrichtung Abfragen der zweiten Softwareinhalte von der Inhaltsdatenbank zur Bereitstellung für die Ultraschalldiagnosevorrichtung, Updaten der Punktinformationen bezüglich der Punktdaten **durch** Subtrahieren eines zweiten vorbestimmten Punkts von den Punktdaten für das Mitglied, welches die Softwareinhalte heruntergeladen hat.

6. Verfahren nach Anspruch 5, wobei das System zum Bereitstellen von Inhalten eine Punktdatenbank zum Speichern von Informationen bezüglich Punktdaten für jedes der in dem System zum Bereitstellen von Inhalten registrierten Mitglieder aufweist, und wobei der Schritt c) Folgendes aufweist:
c1) Auffordern der Ultraschalldiagnosevorrichtung, die ersten Softwareinhalte hochzuladen;
c2) Speichern der von der Ultraschalldiagnosevorrichtung hochgeladenen ersten Softwareinhalte in der Inhaltsdatenbank;
c3) Addieren von vorbestimmten Hochladepunkten zum Hochladen der ersten Softwareinhalte zu den in der Punktdatenbank gespeicherten Punkten.

7. Verfahren nach Anspruch 5, wobei das System zum Bereitstellen von Inhalten eine Punktdatenbank zum Speichern von Informationen bezüglich jedem der in dem System zum Bereitstellen von Inhalten registrierten Mitglieder aufweist, und wobei der Schritt d) Folgendes aufweist:
d1) Erzeugen einer Liste von in der Inhaltsdatenbank gespeicherten Softwareinhalten;
d2) Bereitstellen der Liste für die Ultraschalldiagnosevorrichtung;
d3) Empfangen von Inhaltsauswahlinformationen von der Ultraschalldiagnosevorrichtung;
d4) Abfragen von Softwareinhalten, welche den Inhaltsauswahlinformationen von der Inhaltsdatenbank entsprechen, um die Inhalte der Ultraschalldiagnosevorrichtung zur Verfügung zu stellen; und
d5) Subtrahieren vorbestimmter Downloadpunkte zum Herunterladen der Inhalte von den in der Punktdatenbank gespeicherten Punkten.

## Revendications

1. Système de fourniture de contenus (100) accessible par un dispositif de diagnostic à ultrasons (200) à travers un réseau de communication (300), comprenant une base de données (110) et un serveur (120),
**caractérisé en ce que** :
la base de données (110) est configurée pour stocker une pluralité de contenus logiciels incluant une mise à jour logicielle pour la mise à jour du logiciel de commande et d'exploitation du dispositif de diagnostic à ultrasons (200), des informations sur les membres enregistrés auprès dudit système de fourniture de contenus (100), et des informations sur des données de points pour chacun des membres ; et
le serveur (120) est configuré pour recevoir un premier contenu logiciel pour le dispositif de diagnostic à ultrasons (200) que le dispositif de diagnostic à ultrasons (200) télécharge, et pour stocker le contenu logiciel dans ladite base de données (110), ledit serveur (120) étant en outre configuré pour mettre à jour les informations sur les points en ajoutant un premier point prédéterminé aux données de points pour le membre qui a téléchargé le contenu logiciel, et sensible à une demande de téléchargement d'un second contenu logiciel provenant du dispositif de diagnostic à ultrasons (200) pour récupérer le second contenu logiciel de ladite base de données (110) et le fournir au dispositif de diagnostic à ultrasons (200), le serveur (120) étant en outre configuré pour mettre à jour les informations de points en soustrayant un second point prédéterminé des données de points pour le membre qui a téléchargé le second contenu logiciel.

2. Système de fourniture de contenus de la revendication 1, dans lequel ladite base de données (120) comprend :
une base de données de membres (111) pour stocker les informations sur les membres enregistrés auprès du système de fourniture de contenus ;
une base de données de contenus (112) pour stocker les premier et second contenus logiciels ; et
une base de données de points (113) pour stocker des informations sur les données de points pour chacun des membres, qui sont nécessaires pour télécharger le second contenu logiciel, et dans laquelle les points sont accumulés par téléchargement du premier contenu logiciel ou d'achat de points supplémentaires, au comptant ou par cartes de crédit.

3. Système de fourniture de contenus de la revendication 2, dans lequel ledit serveur (120) génère une liste des contenus logiciels stockés dans ladite base de données de contenus (112) afin de la fournir au dispositif de diagnostic à ultrasons (200), récupère le contenu logiciel correspondant aux informations de sélection de contenu dans ladite base de données de contenus (112) afin de le fournir au dispositif de diagnostic à ultrasons (200) lorsqu'il reçoit des informations de sélection de contenu émanant dudit dispositif de diagnostic à ultrasons (200), et soustrait des points de téléchargement prédéterminés des points stockés dans ladite base de données de points (113), pour le téléchargement du contenu logiciel.

4. Système de fourniture de contenus de la revendication 2, dans lequel ledit serveur (120) stocke le premier contenu logiciel téléchargé à partir du dispositif de diagnostic à ultrasons (200) dans ladite base de données de contenus en réponse à une demande de téléchargement provenant du dispositif de diagnostic à ultrasons (200) et ajoute des points de téléchargement prédéterminés pour le téléchargement de contenus logiciels aux points stockés dans ladite base de données de points (113).

5. Procédé pour fournir des contenus en utilisant un système de fourniture de contenus interconnecté avec un dispositif de diagnostic à ultrasons à travers un réseau de communication,
**caractérisé par** les étapes :
(a) d'agencement d'une base de données de contenus pour stocker une pluralité de contenus logiciels incluant une mise à jour logicielle pour la mise à jour d'un logiciel de commande et d'exploitation du dispositif de diagnostic à ultrasons dans le système de fourniture de contenus, d'information sur les membres enregistrés auprès dudit système de fourniture de contenu, et d'information sur des données de points pour chacun des membres ;
(b) de connexion du dispositif de diagnostic à ultrasons au système de fourniture de contenus à travers le réseau de communication ;
(c) de réception d'un premier contenu logiciel pour le dispositif de diagnostic à ultrasons, le dispositif de diagnostic à ultrasons téléchargeant et stockant le premier contenu logiciel dans la base de données de contenus, et de mise à jour des informations de points sur les données de points en ajoutant un premier point prédéterminé aux données de points pour le membre qui a téléchargé le premier contenu logiciel ;
(d) en présence d'une demande de téléchargement pour le second contenu logiciel émanant du dispositif de diagnostic à ultrasons, de récupération du second contenu logiciel dans la base de données de contenus pour le fournir au dispositif de diagnostic à ultrasons, de mise à jour des informations de points sur les données de points en soustrayant un deuxième point prédéterminé des données de points pour le membre qui a téléchargé le second contenu logiciel.

6. Procédé de la revendication 5, dans lequel le système de fourniture de contenus comprend une base de données de points pour stocker des informations sur des données de points pour chacun des membres enregistrés auprès du système de fourniture de contenus, et dans lequel l'étape (c) comprend :
(c1) la demande au dispositif de diagnostic à ultrasons de télécharger le premier contenu logiciel ;
(c2) le stockage du premier contenu logiciel téléchargé en provenance du dispositif de diagnostic à ultrasons dans la base de données de contenus ;
(c3) l'addition des points de téléchargement prédéterminés pour le téléchargement du premier contenu logiciel aux points stockés dans la base de données de points.

7. Procédé de la revendication 5, dans lequel le système de fourniture de contenus comprend une base de données de points pour stocker des informations sur les points de chacun des membres enregistrés auprès du système de fourniture de contenus, et dans lequel l'étape (d) comprend :
(d1) la génération d'une liste des contenus logiciels stockés dans la base de données de contenus ;
(d2) la fourniture de la liste au dispositif de diagnostic à ultrasons ;
(d3) la réception des informations de sélection de contenus en provenance du dispositif de diagnostic à ultrasons ;
(d4) la récupération du contenu logiciel correspondant aux informations de sélection de contenus dans la base de données de contenus pour fournir le contenu au dispositif de diagnostic à ultrasons ; et
(d5) la soustraction des points de téléchargement pour le téléchargement de contenu des points stockés dans la base de données de points.
